# EUROPÄISCHE PATENTSCHRIFT

(11) **EP 2 658 841 B1**
(45) Veröffentlichungstag und Bekanntmachung des Hinweises auf die Patenterteilung: **20.09.2017**
(21) Anmeldenummer: 11802742.4
(22) Anmeldetag: 23.12.2011
(51) Int. Cl.: C07D 231/20, C07C 315/04

(54) **VERFAHREN ZUR HERSTELLUNG VON 3-ALKYLSULFINYLBENZOYLDERIVATEN**
PROCESS FOR PREPARING 3-ALKYLSULFINYLBENZOYL DERIVATIVES
PROCÉDÉ DE PRÉPARATION DE DÉRIVÉS DE D'ALKYL-3-SULFINYLBENZOYLE

(30) Priorität: 28.12.2010 EP 10197151
(43) Veröffentlichungstag der Anmeldung: 06.11.2013
(73) Patentinhaber: Bayer Intellectual Property GmbH, 40789 Monheim (DE)
(72) Erfinder: FORD, Mark, James, 61389 Schmitten (DE); SCHMIDT, Jan, Peter, 69263 Lyon (FR); KOHLHEPP, Helmut, 36137 Grossenlüder/Bimbach (DE)
(74) Vertreter: BIP Patents
(86) Internationale Anmeldenummer: PCT/EP2011/073906
(87) Internationale Veröffentlichungsnummer: WO 2012/089644

(56) Entgegenhaltungen:
- WO-A1-96/26200
- WO-A1-2005/122768
- US-A1- 2008 305 956

## Beschreibung

Die Erfindung betrifft das technische Gebiet der Herstellung von Pflanzenschutzmitteln.

Aus verschiedenen Schriften ist bereits bekannt, daß bestimmte 3-Alkylsulfinylbenzoylderivate herbizide Eigenschaften besitzen. So werden in EP 2 167 473 A1, EP 2 127 521 A1, WO 2008/125214 A1, WO 2009/149806 A1 und WO 2011/012246 A1 jeweils 3-Alkylsulfinylbenzoylpyrazole als Herbizide beschrieben. In WO 2011/012247 A1 werden jeweils 3-Alkylsulfinylbenzoylcyclohexandione als Herbizide beschrieben. In WO 2011/035874 A1 werden N-(1,2,5-Oxadiazol-3-yl)benzamide beschrieben, die in 3-Position des Phenylrings unter anderem eine 3-Alkylsulfinylgruppe tragen.
Die in diesen Schriften genannten Herstellverfahren betreffen beispielsweise ein in Schema 1 gezeigtes Kondensationsverfahren, bei dem eine 3-Alkylsulfinylbenzoesäure der Formel (I) mit einer Verbindung der Formel (II) in Gegenwart eines wasserentziehenden Mittels, wie *N*-(3-Dimethylaminopropyl)-*N*¹-ethylcarbodiimid-Hydrochlorid (EDCI), und gegebenenfalls eines Cyanids zu dem gewünschten 3-Alkylsulfinylbenzoylderivat der Formel (III) umgesetzt wird.

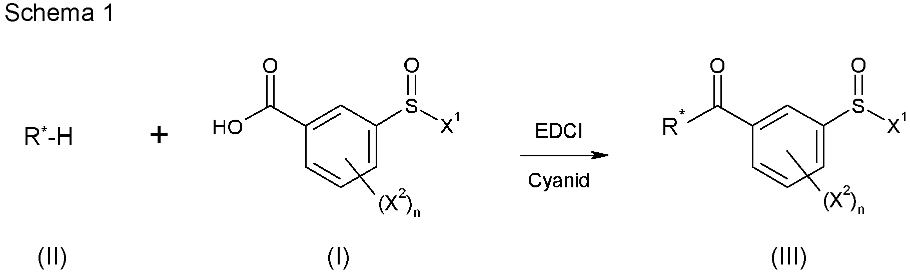

Darin haben die Reste folgende Bedeutungen: X¹ bedeutet Alkyl, X² steht für verschiedene Reste, R*-H ist jeweils ein gegebenenfalls substituiertes 5-Hydroxypyrazol oder Cyclohexandion, n bedeutet 0, 1 oder 2.

Weiterhin wird in einigen der oben genannten Schriften ein in gemäß Schema 2 gezeigtes Herstellverfahren genannt, bei dem in einem ersten Schritt die 3-Alkylsulfinylbenzoesäure der Formel (I) mit beispielsweise Thionylchlorid zum 3-Alkylsulfinylbenzoesäure-chlorid der Formel (Ia) und dieses dann mit einer Verbindung der Formel (II) in Gegenwart einer Base und eines Cyanids zu dem gewünschten 3-Alkylsulfinylbenzoylderivat der Formel (III) umgesetzt wird.

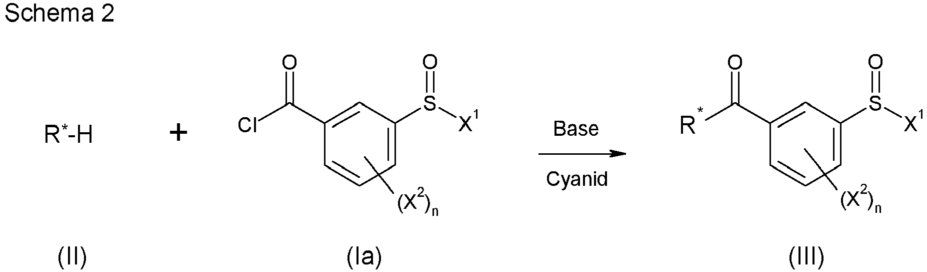

Die aus diesen Schriften bekannten Verfahren weisen jedoch einige Nachteile auf. So werden in dem Verfahren gemäß Schema 1 sehr teuere und toxische wasserentziehende Mittel verwendet. Da diese Mittel nicht katalytisch, sonder äquimolar eingesetzt werden, entstehen zudem große Mengen an Abfallprodukten. Das in Schema 2 gezeigte Verfahren weist den Nachteil auf, dass es nur zu einer geringen Ausbeute des gewünschten 3-Alkylsulfinylbenzoylderivats der Formel (III) führt. Verantwortlich für die geringe Ausbeute ist die dem Fachmann bekannte mangelnde chemische Stabilität der 3-Alkylsulfinylbenzoesäurechloride der Formel (Ia), die sehr schnell zu den entsprechenden 3-Alkylsulfenylbenzoylderivaten reduziert werden. Weiterhin ist beispielsweise aus "Nachrichten aus der Chemie, Technik und Laboratorium", 1983, 31, 11, 892-896, bekannt, dass beim Versuch der Herstellung von Verbindungen der Formel (Ia) mit Hilfe von Chlorierungsmitteln üblicherweise das Chlorierungsmittel ein instabiles Sulfiniumchlorid (Q) erzeugt, welches nicht zum gewünschten Produkt (III) führt, sondern in einer Pummerer Umlagerung zur Verbindung der Formel (W) führt (Schema 2b).

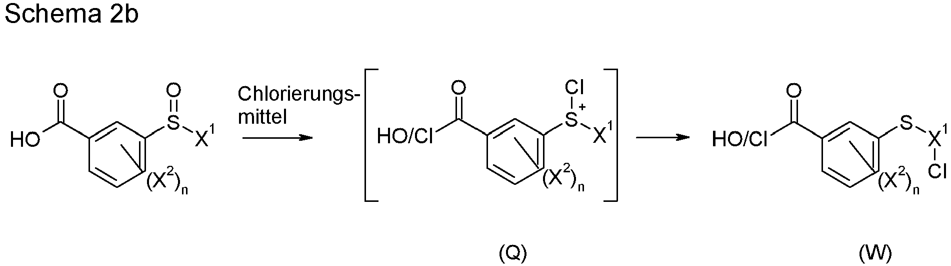

Aufgabe der vorliegenden Erfindung ist die Bereitstellung eines Verfahrens zur Herstellung von 3-Alkylsulfinylbenzoylderivaten, welches die Nachteile der aus dem Stand der Technik bekannten Verfahren überwindet.

Es wurde nun gefunden, dass bestimmte 3-Alkylsulfinylbenzoylderivate sich kostengünstig und in hohen Ausbeuten herstellen lassen durch Reaktion von 3-Alkylsulfinylbenzoesäuren und Verbindungen aus der Gruppe bestehend aus jeweils unsubstituierten oder substituierten 5-Hydroxypyrazolen, Cyclohexandionen, 4-Amino-1,2,5-oxadiazolen, 5-Amino-1H-1,2,4-triazolen und 5-Amino-1H-tetrazolen in Gegenwart eines Chlorierungsmittels, einer Base und gegebenenfalls einer CyanidQuelle.

Ein Gegenstand vorliegender Erfindung ist somit ein Verfahren zur Herstellung von 3-Alkylsulfinylbenzoylderivaten der Formel (IIIa) durch Reaktion von 3-Alkylsulfinylbenzoesäuren der Formel (Ib) mit Verbindungen der Formel (II) in Gegenwart eines Chlorierungsmittels und einer Base. 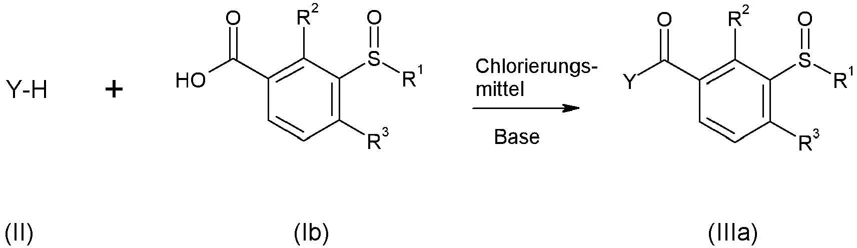 worin die Reste, Symbole und Indices folgende Bedeutungen haben:
R¹ bedeutet jeweils durch s Reste aus der Gruppe bestehend aus Halogen, (C₁-C₂)-Alkoxy und Halogen-(C₁-C₂)-Alkyl substituiertes (C₁-C₈)-Alkyl, (C₃-C₈)-Cycloalkyl oder Benzyl,
R² bedeutet Wasserstoff, Mercapto, Nitro, Halogen, Cyano, Rhodano, (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₃-C₆)-Halogenalkinyl, (C₃-C₆)-Cycloalkyl, OR⁴, OCOR⁴, OSO₂R⁴, S(O)ₙR⁴, SO₂OR⁴, SO₂N(R⁴)₂, NR⁴SO₂R⁴, NR⁴COR⁴, (C₁-C₆)-Alkyl-S(O)ₙR⁴, (C₁-C₆)-Alkyl-OR⁴, (C₁-C₆)-Alkyl-OCOR⁴, (C₁-C₆)-Alkyl-OSO₂R⁴, (C₁-C₆)-Alkyl-SO₂OR⁴, (C₁-C₆)-Alkyl-SO₂N(R⁴)₂ oder (C₁-C₆)-Alkyl-NR⁴COR⁴,
R³ bedeutet Fluor, Chlor, Brom, Iod, Nitro, CF₃ oder die Gruppe SO₂R,
R⁴ bedeutet Wasserstoff, (C₁-C₆)-Alkyl, (C₂-C₆)-Alkenyl, (C₂-C₆)-Alkinyl, (C₃-C₆)-Cycloalkyl, Phenyl oder Phenyl-(C₁-C₆)-alkyl, wobei die sechs letztgenannten Reste durch s Reste der Gruppe Hydroxy, Mercapto, Amino, Cyano, Nitro, Rhodano, OR⁵, SR⁵, N(R⁵)₂, NOR⁵, OCOR⁵, SCOR⁵, NR⁵COR⁵, CO₂R⁵, COSR⁵, CON(R⁵)₂, (C₁-C₄)-Alkyliminooxy, (C₁-C₄)-Alkylcarbonyl, (C₁-C₄)-Alkoxy-(C₂-C₆)-alkoxycarbonyl und (C₁-C₄)-Alkylsulfonyl substituiert sind,
R⁵ bedeutet Wasserstoff, (C₁-C₆)-Alkyl, (C₂-C₆)-Alkenyl oder (C₂-C₆)-Alkinyl,
R bedeutet (C₁-C₄)-Alkyl,
n bedeutet 0, 1 oder 2,
s bedeutet 0, 1, 2 oder 3,
Y bedeutet einen Rest Y1, Y2, Y3, Y4, Y5 oder Y6 : 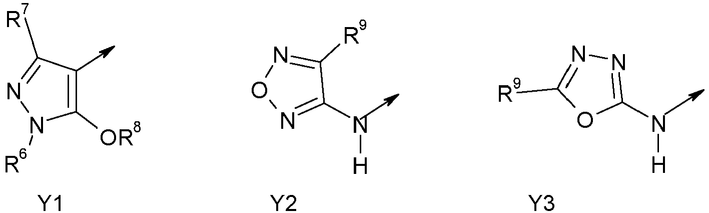 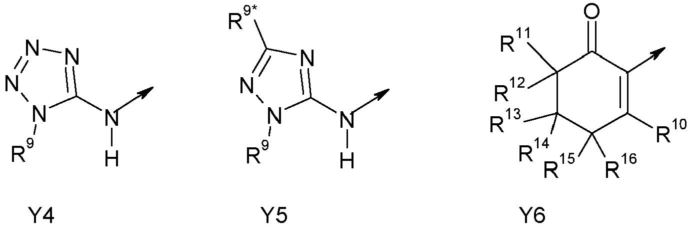
R⁶ bedeutet (C₁-C₄)-Alkyl,
R⁷ bedeutet Wasserstoff oder (C₁-C₄)-Alkyl,
R⁸ bedeutet Wasserstoff, (C₁-C₆)-Alkylsulfonyl, (C₁-C₄)-Alkoxy-(C₁-C₆)-alkylsulfonyl, oder jeweils durch s Reste aus der Gruppe bestehend aus Halogen, (C₁-C₄)-Alkyl und (C₁-C₄)-Alkoxy substituiertes Phenylsulfonyl, Thiophenyl-2-sulfonyl, Benzoyl, Benzoyl-(C₁-C₆)-alkyl oder Benzyl,
R⁹ bedeutet Wasserstoff, (C₁-C₆)-Alkyl, (C₃-C₇)-Cycloalkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, CH₂R¹⁸ oder durch s Reste aus der Gruppe Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl substituiertes Phenyl,
R^{9*} bedeutet Wasserstoff, Nitro, Halogen, Cyano, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, (C₃-C₆)-Cycloalkyl, (C₃-C₆)-Cycloalkenyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, COR¹, COOR¹,OCOOR¹, NR¹COOR¹, C(O)N(R¹)₂, NR¹C(O)N(R¹)₂, OC(O)N(R¹)₂, CO(NOR¹)R¹, NR¹SO₂R², NR¹COR¹, OR¹, OSO₂R², S(O)ₙR², SO₂OR¹, SO₂N(R¹)₂ (C₁-C₆)-Alkyl-S(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-CO₂R¹, (C₁-C₆)-Alkyl-CN, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², N(R¹)₂, P(O)(OR⁵)₂, CH₂P(O)(OR⁵)₂, (C₁-C₆)-Alkyl-Phenyl, (C₁-C₆)-Alkyl-Heteroaryl, (C₁-C₆)-Alkyl-Heterocyclyl, Phenyl, Heteroaryl oder Heterocyclyl, wobei die letzten 6 Reste jeweils durch s Reste aus der Gruppe Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl und Cyanomethyl substituiert sind, und wobei Heterocyclyl 0 bis 2 Oxogruppen trägt,
R¹⁰ bedeutet Hydroxy oder SR¹⁷,
R¹¹ und R¹⁶ bedeuten unabhängig voneinander Wasserstoff oder (C₁-C₄)-Alkyl, oder die Reste R¹¹ und R¹⁶ bilden zusammen die Einheit Z, die ein Sauerstoff- oder Schwefelatom oder ein bis vier Methylengruppen darstellt,
R¹² und R¹⁵ bedeuten unabhängig voneinander Wasserstoff oder (C₁-C₄)-Alkyl,
R¹³ und R¹⁴ bedeuten unabhängig voneinander Wasserstoff oder (C₁-C₄)-Alkyl oder bilden gemeinsam mit dem Kohlenstoffatom, an das sie gebunden sind, eine Carbonylgruppe,
R¹⁷ bedeutet (C₁-C₄)-Alkyl, durch s Reste aus der Gruppe bestehend aus Nitro, Cyano, (C₁-C₄)-Alkyl, (C₁-C₄)-Halogenalkyl, (C₁-C₄)-Alkoxy und (C₁-C₄)-Halogen-alkoxy substituiertes Phenyl oder partiell oder vollständig halogeniertes Phenyl,
R¹⁸ bedeutet Acetoxy, Acetamido, N-Methylacetamido, Benzoyloxy, Benzamido, N-Methylbenzamido, Methoxycarbonyl, Ethoxycarbonyl, Benzoyl, Methylcarbonyl, Piperidinylcarbonyl, Morpholinylcarbonyl, Trifluormethylcarbonyl, Aminocarbonyl, Methylaminocarbonyl, Dimethylaminocarbonyl, (C₁-C₆)-Alkoxy, (C₃-C₆)-Cycloalkyl oder durch s Reste aus der Gruppe Methyl, Ethyl, Methoxy, Trifluormethyl und Halogen substituiertes Phenyl.

In den oben genannten Resten Y1 bis Y6 bedeutet der Pfeil jeweils die Verknüpfung zum restlichen Molekül.

Die durch das erfindungsgemäße Verfahren herstellbare Verbindungen der Formel (IIIa) weisen somit in Abhängigkeit von der Bedeutung des Restes Y die Strukturen der Formeln (IIIa1), (IIIa2), (IIIa3), (IIIa4), (IIIa5) und (IIIa6) auf:

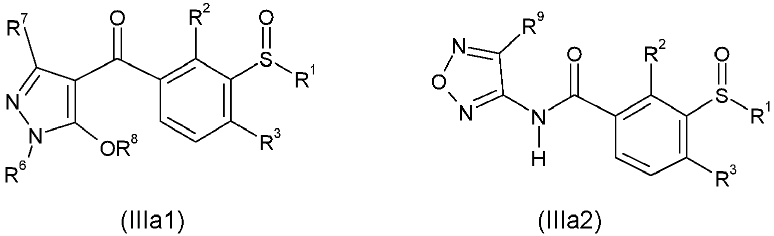

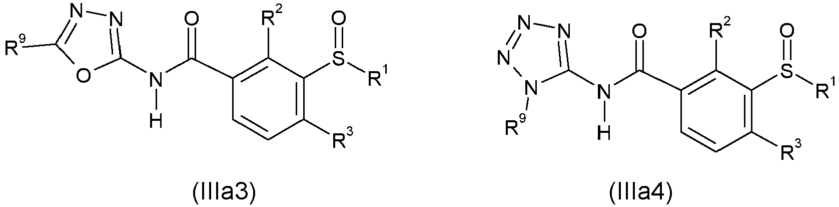

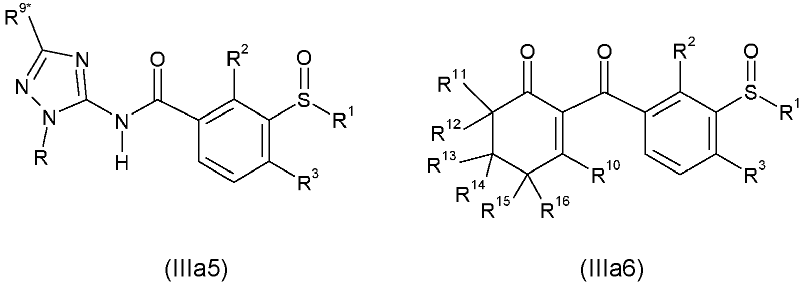

Ein besonderer Vorteil des erfindungsgemäßen Verfahrens ist auch die Tatsache, dass es in einer einstufigen Reaktion durchgeführt werden kann, ohne dass weitere Zwischenstufen isoliert werden müssen.

Ein weiterer Vorteil des erfindungsgemäßen Verfahrens ist auch die Tatsache, dass die stereochemische Information der Verbindungen der Formel (Ib) während der Reaktion zu den Verbindungen der Formel (IIIa) nicht verloren geht, d.h. es findet keine Racemisierung statt, insbesondere nicht an der Sulfinylgruppe S(=O)R¹.

In der Formel (I) und allen nachfolgenden Formeln können Alkylreste mit mehr als zwei Kohlenstoffatomen geradkettig oder verzweigt sein. Alkylreste bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl. Halogen steht für Fluor, Chlor, Brom oder Iod. Tosyl bedeutet 4-Methylphenylsulfonyl.

Heterocyclyl bedeutet einen gesättigten, teilgesättigten oder vollständig ungesättigten cyclischen Rest, der 3 bis 6 Ringatome enthält, von denen 1 bis 4 aus der Gruppe Sauerstoff, Stickstoff und Schwefel stammen, und der zusätzlich durch einen Benzoring annelliert sein kann. Beispielsweise steht Heterocyclyl für Piperidinyl, Pyrrolidinyl, Tetrahydrofuranyl, Dihydrofuranyl und Oxetanyl,

Heteroaryl bedeutet einen aromatischen cyclischen Rest, der 3 bis 6 Ringatome enthält, von denen 1 bis 4 aus der Gruppe Sauerstoff, Stickstoff und Schwefel stammen, und der zusätzlich durch einen Benzoring annelliert sein kann. Beispielsweise steht Heteroaryl für Benzimidazol-2-yl, Furanyl, Imidazolyl, Isoxazolyl, Isothiazolyl, Oxazolyl, Pyrazinyl, Pyrimidinyl, Pyridazinyl, Pyridinyl, Benzisoxazolyl, Thiazolyl, Pyrrolyl, Pyrazolyl, Thiophenyl, 1,2,3-Oxadiazolyl, 1,2,4-Oxadiazolyl, 1,2,5-Oxadiazolyl, 1,3,4-Oxadiazolyl, 1,2,4-Triazolyl, 1,2,3-Triazolyl, 1,2,5-Triazolyl, 1,3,4-Triazolyl, 1,2,4-Triazolyl, 1,2,4-Thiadiazolyl, 1,3,4-Thiadiazolyl, 1,2,3-Thiadiazolyl, 1,2,5-Thiadiazolyl, 2H-1,2,3,4-Tetrazolyl, 1H-1,2,3,4-Tetrazolyl, 1,2,3,4-Oxatriazolyl, 1,2,3,5-Oxatriazolyl, 1,2,3,4-Thiatriazolyl und 1,2,3,5-Thiatriazolyl.

Ist eine Gruppe mehrfach durch Reste substituiert, so ist darunter zu verstehen, daß diese Gruppe durch ein oder mehrere gleiche oder verschiedene der genannten Reste substituiert ist.

In allen nachfolgend genannten Formeln haben die Substituenten, Symbole und Indices, sofern nicht anders definiert, dieselbe Bedeutung wie unter den vorstehend genannten Formeln (Ib), (II), (IIIa), (IIIa1), (IIIa2), (IIIa3), (IIIa4), (IIIa5), Y1, Y2, Y3, Y4 und Y5 beschrieben.

In dem erfindungsgemäßen Verfahren haben die Reste, Symbole und Indices vorzugsweise folgende Bedeutung:
R¹ bedeutet (C₁-C₄)-Alkyl,
R² bedeutet Nitro, Halogen, (C₁-C₄)-Alkyl, Trifluormethyl, (C₁-C₄)-Alkoxy, Methylsulfonyl, Methoxymethyl, Methoxymethoxymethyl, Ethoxyethoxymethyl, Ethoxymethoxymethyl, Methoxyethoxymethyl, Methoxypropoxymethyl, Methylsulfonylmethyl, Methylsulfonylethoxymethyl, Methoxyethylsulfonylmethyl, Methylsulfonylethylsulfonylmethyl,
R³ bedeutet Fluor, Chlor, Brom, Iod, CF₃ oder die Gruppe SO₂R,
Y bedeutet einen Rest Y1, Y2, Y3, Y4, Y5 oder Y6 : 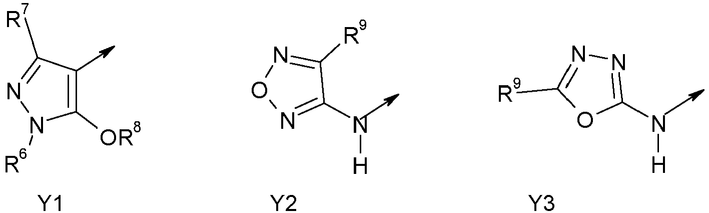 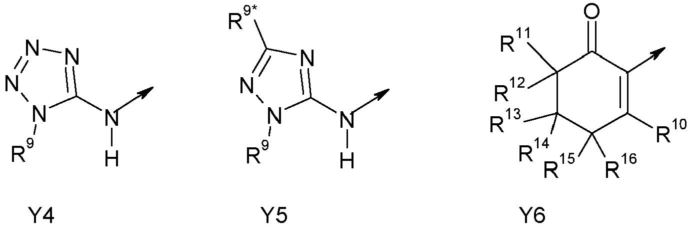
   R⁶ bedeutet Methyl, Ethyl, n-Propyl oder i-Propyl,
   R⁷ bedeutet Wasserstoff, Methyl, Ethyl, n-Propyl oder i-Propyl,
   R⁸ bedeutet Wasserstoff, (C₁-C₃)-Alkylsulfonyl, (C₁-C₂)-Alkoxy-(C₁-C₄)-alkylsulfonyl, oder jeweils durch s Methylgruppen substituiertes Phenylsulfonyl, Thiophenyl-2-sulfonyl, Benzoyl, Benzoyl-(C₁-C₆)-alkyl oder Benzyl,
   s bedeutet 0, 1, 2 oder 3,
   R⁹ bedeutet (C₁-C₆)-Alkyl, (C₃-C₇)-Cycloalkyl, Halogen-(C₁-C₆)-alkyl, (C₃-C₇)-Cycloalkylmethyl, Methoxycarbonylmethyl, Ethoxycarbonylmethyl, Acetylmethyl, Methoxymethyl, oder durch s Reste aus der Gruppe Methyl, Methoxy, Trifluormethyl und Halogen substituiertes Phenyl oder Benzyl,
   R^{9*} bedeutet Wasserstoff, (C₁-C₆)-Alkyl, Halogen-(C₁-C₆)-alkyl, (C₃-C₆)-Cycloalkyl, COR¹, OR¹, COOR¹ oder (C₁-C₆)-Alkyl-Phenyl,
   R¹⁰ bedeutet Hydroxy,
   R¹¹ und R¹⁶ bedeuten unabhängig voneinander Wasserstoff oder (C₁-C₄)-Alkyl, oder die Reste R¹¹ und R¹⁶ bilden zusammen eine Methylen- oder Ethylengruppe,
   R¹² und R¹⁵ bedeuten unabhängig voneinander Wasserstoff, Methyl oder Ethyl,
   R¹³ und R¹⁴ bedeuten unabhängig voneinander Wasserstoff, Methyl oder Ethyl.

Das erfindungsgemäße Verfahren wird in der Regel so durchgeführt, dass die Verbindungen der Formeln (Ib) und (II) in einem organischen Lösungsmittel mit einem Chlorierungsmittel in Anwesenheit einer Base umgesetzt werden. Je nach Reaktivität der Verbindungen der Formeln (Ib) und (II) kann es zweckmäßig sein, das erfindungsgemäße Verfahren auch in Anwesenheit einer Cyanidquelle durchzuführen.

Das erfindungsgemäße Verfahren kann in Abhängigkeit der Reaktivität der eingesetzten Reaktionspartner in einem weiten Temperaturbereich durchgeführt werden. Üblicherweise wird es bei einer Temperatur von - 100 bis - 5°C, vorzugsweise bei einer Temperatur von - 80 bis -15°C, besonders bevorzugt bei einer Temperatur von - 50 bis - 20°C durchgeführt.

Als Chlorierungsmittel eignen sich Thionylchlorid, Phosphoroxychlorid, Oxalylchlorid und Phosgen. Bevorzugte Chlorierungsmittel sind Thionylchlorid und Phosgen. Besonders bevorzugtes Chlorierungsmittel ist Thionylchlorid.

Als Basen werden üblicherweise schwache Basen, d.h. solche mit einem pK_{B}-Wert von grösser als 4,75 eingesetzt. Bevorzugte Basen sind organische Amine. Besonders bevorzugte Basen sind Pyridin, Alkylpyridine wie 3-Methylpyridin und Dialkylpyridine wie 3,5-Dimethylpyridin oder 2-Methyl-5-ethylpyridin.

Als Lösungsmittel eignen sich aprotische und dipolar aprotische Lösungsmittel, wie Alkansäurealkylester, Chloralkane, Chloraromaten, Chloralkylaromaten, Alkylnitrile und Arylnitrile. Bevorzugte Lösungsmittel sind Ethylacetat, Propylacetat, Isopropylacetat, Butylacetat, Dichlormethan, Dichlorethan, Chlortoluol, Acetonitril, Butyronitril und Benzonitril. Besonders bevorzugte Lösungsmittel sind Ethylacetat, Dichlormethan und Dichlorethan.

Als Cyanidquelle werden üblicherweise solche Cyanidverbindungen eingesetzt, die in der Lage sind, in den oben genannten Lösungsmitteln Cyanid abzuspalten. Bevorzugte Cyanidquellen sind Acetoncyanhydrin und Alkalicyanide, wie Kaliumcyanid und Natriumcyanid. Besonders bevorzugte Cyanidquelle ist Acetoncyanhydrin.

In dem erfindungsgemäßen Verfahren werden die Verbindungen der Formeln (Ib) und (II) üblicherweise in einem stöchiometrischen Verhältnis eingesetzt. Das Chlorierungsmittel wird üblicherweise in einem Verhältnis von 1:1 bis 1,5:1 zur Verbindung der Formel (Ib) eingesetzt. Die Base wird üblicherweise in einem Verhältnis von 1:1 bis 6:1 zur Verbindung der Formel (Ib) eingesetzt. Die Cyanidquelle wird, sofern erforderlich, üblicherweise katalytisch, d.h. mit etwa 1 bis 10-Molprozent in Bezug auf Verbindung der Formel (Ib) eingesetzt.

In der Regel verläuft das erfindungsgemäße Verfahren auch bei tiefen Temperaturen schnell und in hohen Ausbeuten ab. Die nachfolgenden Beispiele erläutern das erfindungsgemäße Verfahren näher.
1a. Herstellung von (5-Hydroxy-1-methyl-1H-pyrazol-4-yl)[2-methoxy-3-(methylsulfinyl)-4-(trifluormethyl)phenyl]methanon 3.0 g 2-Methoxy-3-methylsulfinyl-4-trifluormethylbenzoesäure und 1.02 g 3-Hydroxy-2-methylpyrazol werden in 30 ml Ethylacetat und 4.21 ml Pyridin unter einer Inertatmosphäre suspendiert und unter Rühren auf -20°C gekühlt. Dann werden 1.24 g Thionylchlorid so langsam zugetropft, dass die Temperatur stets unter -15°C bleibt. Nach der vollständigen Zugabe des Thionylchlorids wird noch 20 Minuten gerührt und auf -10°C erwärmt. Dann werden 10 ml kaltes Wasser zugegeben und auf 0°C erwärmt. Nach Zugabe von 40 ml Heptan wird die Suspension filtriert, mit 20 ml Heptan, 10 ml Wasser und nochmals mit 20 ml Heptan gewaschen. Nach Trocknung erhält man 3.32 g (5-Hydroxy-1-methyl-1H-pyrazol-4-yl)[2-methoxy-3-(methylsulfinyl)-4-(trifluormethyl)phenyl]methanon (84% Ausbeute).
1b. Herstellung von (5-Hydroxy-1-methyl-1H-pyrazol-4-yl)[2-methoxy-3-(methylsulfinyl)-4-(trifluormethyl)phenyl]methanon 3.0 g 2-Methoxy-3-methylsulfinyl-4-trifluormethylbenzoesäure und 1.02 g 3-Hydroxy-2-methylpyrazol werden in 20 ml Ethylacetat und 5.07 ml 3-Methylpyridin unter einer Inertatmosphäre suspendiert und unter Rühren auf -25°C gekühlt. Dann werden 1.24 g Thionylchlorid so langsam zugetropft, dass die Temperatur stets unter -20°C bleibt. Nach der vollständigen Zugabe des Thionylchlorids wird noch 20 Minuten gerührt. Dann werden 40 µl kaltes Wasser und 20 ml Methylcyclohexan zugegeben. Man lässt bei -25°C noch 20 Minuten rühren. Die Suspension wird filtriert und einmal mit 10 ml Methylcyclohexan und zweimal mit je 10 ml Wasser gewaschen. Nach Trocknung erhält man 3.67 g (5-Hydroxy-1-methyl-1H-pyrazol-4-yl)[2-methoxy-3-(methylsulfinyl)-4-(trifluormethyl)phenyl]methanon (86,5% Ausbeute).
2. Herstellung von [3-(Ethylsulfinyl)-2-methyl-4-(methylsulfonyl)phenyl](5-hydroxy-1-methyl-1H-pyrazol-4-yl)methanon 305 mg 2-Methyl-3-ethylsulfinyl-4-methylsulfonylbenzoesäure und 106 mg 3-Hydroxy-2-methylpyrazol werden in 2 ml Ethylacetat und 407 mg Pyridin unter einer Inertatmosphäre suspendiert und unter Rühren auf -25°C gekühlt. Dann werden 129 mg Thionylchlorid so langsam zugetropft, dass die Temperatur stets unter -20°C bleibt. Nach der vollständigen Zugabe des Thionylchlorids wird noch 20 Minuten gerührt. Es werden weitere 27 mg 3-Hydroxy-2-methylpyrazol zugegeben und 42 mg Thionylchlorid so langsam zugetropft, dass die Temperatur stets unter -25°C bleibt. Dann werden 4 µl kaltes Wasser zugegeben und auf 0°C erwärmt. Nach Trennung der organischen von der wässrigen Phase wird die wässrige Phase zweimal mit je 5 ml Ethylacetat extrahiert. Die vereinigten organischen Phasen werden getrocknet und man erhält 370 mg [3-(Ethylsulfinyl)-2-methyl-4-(methylsulfonyl)phenyl](5-hydroxy-1-methyl-1H-pyrazol-4-yl)methanon (91 % Ausbeute).
3. Herstellung von (5-Hydroxy-1-methyl-1H-pyrazol-4-yl)[2-methyl-3-(methylsulfinyl)-4-(trifluormethyl)phenyl]methanon 3.0 g 2-Methyl-3-methylsulfinyl-4-trifluormethylbenzoesäure und 1.25 g 3-Hydroxy-2-methylpyrazol werden in 20 ml Ethylacetat und 4.8 g Pyridin unter einer Inertatmosphäre suspendiert und unter Rühren auf -25°C gekühlt. Dann werden 1.51 g Thionylchlorid so langsam zugetropft, dass die Temperatur stets unter -20°C bleibt. Nach der vollständigen Zugabe des Thionylchlorids wird noch 20 Minuten gerührt. Es werden 40 µl kaltes Wasser, 10 ml Ethylacetat und weitere 10 ml Wasser zugegeben und auf 0°C erwärmt. Nach Trennung der organischen von der wässrigen Phase wird die wässrige Phase zweimal mit je 5 ml Ethylacetat extrahiert. Die vereinigten organischen Phasen werden getrocknet und man erhält 3.69 g (5-Hydroxy-1-methyl-1H-pyrazol-4-yl)[2-methyl-3-(methylsulfinyl)-4-(trifluormethyl)phenyl]methanon (90.7% Ausbeute).
4. Herstellung von 3-[2-Methoxy-3-(methylsulfinyl)-4-(trifluormethyl)benzoyloxy] cyclohex-2-en-1-on 3.0 g 2-Methoxy-3-methylsulfinyl-4-trifluormethylbenzoesäure und 1.68 g 1,3-Cyclohexandion werden in 20 ml Ethylacetat und 5.61 ml Pyridin unter einer Inertatmosphäre suspendiert und unter Rühren auf -25°C gekühlt. Dann werden 1.73 g Thionylchlorid so langsam zugetropft, dass die Temperatur stets unter -20°C bleibt. Nach der vollständigen Zugabe des Thionylchlorids wird noch 20 Minuten gerührt. Es werden 38 µl kaltes Wasser zugegeben und auf 0°C erwärmt. Dann werden 10 ml Ethylacetat und weitere 10 ml Wasser zugegeben. Nach Trennung der organischen von der wässrigen Phase wird die wässrige Phase zweimal mit je 5 ml Ethylacetat extrahiert. Die vereinigten organischen Phasen werden getrocknet und man erhält 3.57 g 3-[2-Methoxy-3-(methylsulfinyl)-4-(trifluormethyl)benzoyloxy] cyclohex-2-en-1-on (85.5% Ausbeute).

## Patentansprüche

1. Verfahren zur Herstellung von 3-Alkylsulfinyl-benzoylderivaten der Formel (IIIa) durch Reaktion von 3-Alkylsulfinylbenzoesäuren der Formel (Ib) mit Verbindungen der Formel (II) in Gegenwart eines Chlorierungsmittels und einer Base und gegebenenfalls einer Cyanidquelle 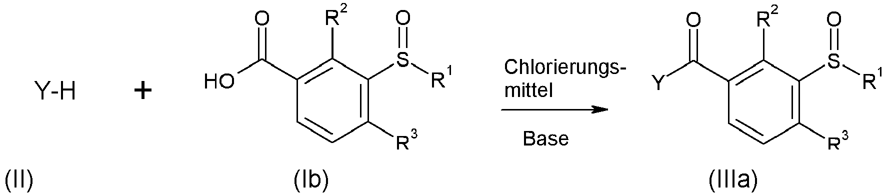 worin die Reste, Symbole und Indices folgende Bedeutungen haben:
R¹ bedeutet jeweils durch s Reste aus der Gruppe bestehend aus Halogen, (C₁-C₂)-Alkoxy und Halogen-(C₁-C₂)-Alkyl substituiertes (C₁-C₈)-Alkyl, (C₃-C₈)-Cycloalkyl oder Benzyl,
R² bedeutet Wasserstoff, Mercapto, Nitro, Halogen, Cyano, Rhodano, (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₃-C₆)-Halogenalkinyl, (C₃-C₆)-Cycloalkyl, OR⁴, OCOR⁴, OSO₂R⁴, S(O)ₙR⁴, SO₂OR⁴, SO₂N(R⁴)₂, NR⁴SO₂R⁴, NR⁴COR⁴, (C₁-C₆)-Alkyl-S(O)ₙR⁴, (C₁-C₆)-Alkyl-OR⁴, (C₁-C₆)-Alkyl-OCOR⁴, (C₁-C₆)-Alkyl-OSO₂R⁴, (C₁-C₆)-Alkyl-SO₂OR⁴, (C₁-C₆)-Alkyl-SO₂N(R⁴)₂ oder (C₁-C₆)-Alkyl-NR⁴COR⁴,
R³ bedeutet Fluor, Chlor, Brom, Iod, Nitro, CF₃ oder die Gruppe SO₂R,
R⁴ bedeutet Wasserstoff, (C₁-C₆)-Alkyl, (C₂-C₆)-Alkenyl, (C₂-C₆)-Alkinyl, (C₃-C₆)-Cycloalkyl, Phenyl oder Phenyl-(C₁-C₆)-alkyl, wobei die sechs letztgenannten Reste durch s Reste der Gruppe Hydroxy, Mercapto, Amino, Cyano, Nitro, Rhodano, OR⁵, SR⁵, N(R⁵)₂, NOR⁵, OCOR⁵, SCOR⁵, NR⁵COR⁵, CO₂R⁵, COSR⁵, CON(R⁵)₂, (C₁-C₄)-Alkyliminooxy, (C₁-C₄)-Alkylcarbonyl, (C₁-C₄)-Alkoxy-(C₂-C₆)-alkoxycarbonyl und (C₁-C₄)-Alkylsulfonyl substituiert sind,
R⁵ bedeutet Wasserstoff, (C₁-C₆)-Alkyl, (C₂-C₆)-Alkenyl oder (C₂-C₆)-Alkinyl,
R bedeutet (C₁-C₄)-Alkyl,
n bedeutet 0, 1 oder 2,
s bedeutet 0, 1, 2 oder 3,
Y bedeutet einen Rest Y1, Y2, Y3, Y4, Y5 oder Y6 : 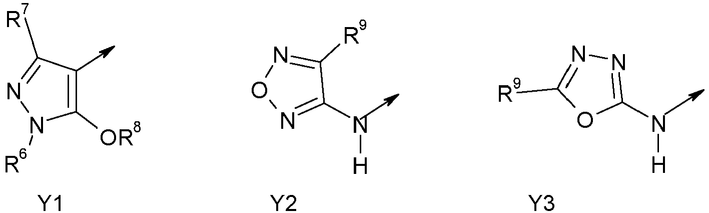 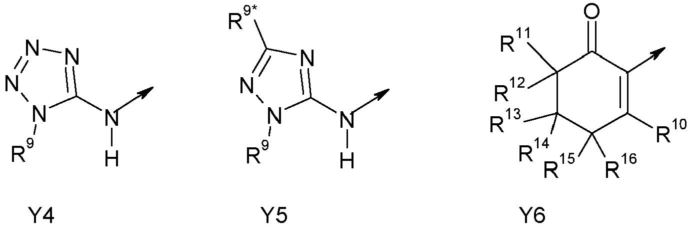
R⁶ bedeutet (C₁-C₄)-Alkyl,
R⁷ bedeutet Wasserstoff oder (C₁-C₄)-Alkyl,
R⁸ bedeutet Wasserstoff, (C₁-C₆)-Alkylsulfonyl, (C₁-C₄)-Alkoxy-(C₁-C₆)-alkylsulfonyl, oder jeweils durch s Reste aus der Gruppe bestehend aus Halogen, (C₁-C₄)-Alkyl und (C₁-C₄)-Alkoxy substituiertes Phenylsulfonyl, Thiophenyl-2-sulfonyl, Benzoyl, Benzoyl-(C₁-C₆)-alkyl oder Benzyl,
R⁹ bedeutet Wasserstoff, (C₁-C₆)-Alkyl, (C₃-C₇)-Cycloalkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, CH₂R¹⁸ oder durch s Reste aus der Gruppe Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl substituiertes Phenyl,
R^{9*} bedeutet Wasserstoff, Nitro, Halogen, Cyano, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, (C₃-C₆)-Cycloalkyl, (C₃-C₆)-Cycloalkenyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, COR¹, COOR¹,OCOOR¹, NR¹COOR¹, C(O)N(R¹)₂, NR¹C(O)N(R¹)₂, OC(O)N(R¹)₂, CO(NOR¹)R¹, NR¹SO₂R², NR¹COR¹, OR¹, OSO₂R², S(O)ₙR², SO₂OR¹, SO₂N(R¹)₂ (C₁-C₆)-Alkyl-S(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-CO₂R¹, (C₁-C₆)-Alkyl-CN, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², N(R¹)₂, P(O)(OR⁵)₂, CH₂P(O)(OR⁵)₂, (C₁-C₆)-Alkyl-Phenyl, (C₁-C₆)-Alkyl-Heteroaryl, (C₁-C₆)-Alkyl-Heterocyclyl, Phenyl, Heteroaryl oder Heterocyclyl, wobei die letzten 6 Reste jeweils durch s Reste aus der Gruppe Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl und Cyanomethyl substituiert sind, und wobei Heterocyclyl 0 bis 2 Oxogruppen trägt,
R¹⁰ bedeutet Hydroxy oder SR¹⁷,
R¹¹ und R¹⁶ bedeuten unabhängig voneinander Wasserstoff oder (C₁-C₄)-Alkyl, oder die Reste R¹¹ und R¹⁶ bilden zusammen die Einheit Z, die ein Sauerstoff- oder Schwefelatom oder ein bis vier Methylengruppen darstellt,
R¹² und R¹⁵ bedeuten unabhängig voneinander Wasserstoff oder (C₁-C₄)-Alkyl,
R¹³ und R¹⁴ bedeuten unabhängig voneinander Wasserstoff oder (C₁-C₄)-Alkyl oder bilden gemeinsam mit dem Kohlenstoffatom, an das sie gebunden sind, eine Carbonylgruppe,
R¹⁷ bedeutet (C₁-C₄)-Alkyl, durch s Reste aus der Gruppe bestehend aus Nitro, Cyano, (C₁-C₄)-Alkyl, (C₁-C₄)-Halogenalkyl, (C₁-C₄)-Alkoxy und (C₁-C₄)-Halogen-alkoxy substituiertes Phenyl oder partiell oder vollständig halogeniertes Phenyl,
R¹⁸ bedeutet Acetoxy, Acetamido, N-Methylacetamido, Benzoyloxy, Benzamido, N-Methylbenzamido, Methoxycarbonyl, Ethoxycarbonyl, Benzoyl, Methylcarbonyl, Piperidinylcarbonyl, Morpholinylcarbonyl, Trifluormethylcarbonyl, Aminocarbonyl, Methylaminocarbonyl, Dimethylaminocarbonyl, (C₁-C₆)-Alkoxy, (C₃-C₆)-Cycloalkyl oder durch s Reste aus der Gruppe Methyl, Ethyl, Methoxy, Trifluormethyl und Halogen substituiertes Phenyl.

2. Verfahren nach Anspruch 1, worin die Reste, Symbole und Indices folgende Bedeutungen haben:
R¹ bedeutet (C₁-C₄)-Alkyl,
R² bedeutet Nitro, Halogen, (C₁-C₄)-Alkyl, Trifluormethyl, (C₁-C₄)-Alkoxy, Methylsulfonyl, Methoxymethyl, Methoxymethoxymethyl, Ethoxyethoxymethyl, Ethoxymethoxymethyl, Methoxyethoxymethyl, Methoxypropoxymethyl, Methylsulfonylmethyl, Methylsulfonylethoxymethyl, Methoxyethylsulfonylmethyl, Methylsulfonylethylsulfonylmethyl,
R³ bedeutet Fluor, Chlor, Brom, Iod, CF₃ oder die Gruppe SO₂R,
Y bedeutet einen Rest Y1, Y2, Y3, Y4, Y5 oder Y6 : 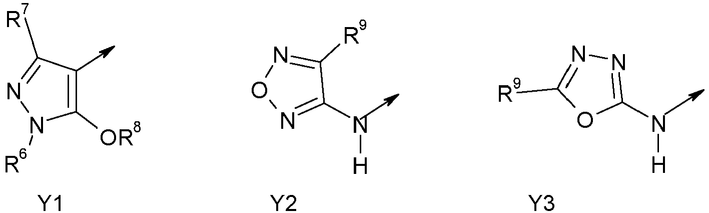 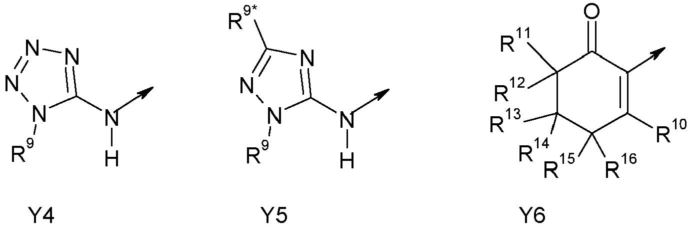
R⁶ bedeutet Methyl, Ethyl, n-Propyl oder i-Propyl,
R⁷ bedeutet Wasserstoff, Methyl, Ethyl, n-Propyl oder i-Propyl,
R⁸ bedeutet Wasserstoff, (C₁-C₃)-Alkylsulfonyl, (C₁-C₂)-Alkoxy-(C₁-C₄)-alkylsulfonyl, oder jeweils durch s Methylgruppen substituiertes Phenylsulfonyl, Thiophenyl-2-sulfonyl, Benzoyl, Benzoyl-(C₁-C₆)-alkyl oder Benzyl,
s bedeutet 0, 1, 2 oder 3,
R⁹ bedeutet (C₁-C₆)-Alkyl, (C₃-C₇)-Cycloalkyl, Halogen-(C₁-C₆)-alkyl, (C₃-C₇)-Cycloalkylmethyl, Methoxycarbonylmethyl, Ethoxycarbonylmethyl, Acetylmethyl, Methoxymethyl, oder durch s Reste aus der Gruppe Methyl, Methoxy, Trifluormethyl und Halogen substituiertes Phenyl oder Benzyl,
R^{9*} bedeutet Wasserstoff, (C₁-C₆)-Alkyl, Halogen-(C₁-C₆)-alkyl, (C₃-C₆)-Cycloalkyl, COR¹, OR¹, COOR¹ oder (C₁-C₆)-Alkyl-Phenyl,
R¹⁰ bedeutet Hydroxy,
R¹¹ und R¹⁶ bedeuten unabhängig voneinander Wasserstoff oder (C₁-C₄)-Alkyl, oder die Reste R¹¹ und R¹⁶ bilden zusammen eine Methylen- oder Ethylengruppe,
R¹² und R¹⁵ bedeuten unabhängig voneinander Wasserstoff, Methyl oder Ethyl,
R¹³ und R¹⁴ bedeuten unabhängig voneinander Wasserstoff, Methyl oder Ethyl.

3. Verfahren nach Anspruch 1 oder 2, wobei die Reaktion bei einer Temperatur von - 80 bis -15°C durchgeführt wird.

4. Verfahren nach einem der Ansprüche 1 bis 3, wobei die Reaktion bei einer Temperatur von - 50 bis - 20°C durchgeführt wird.

5. Verfahren nach einem der Ansprüche 1 bis 4, wobei das Chlorierungsmittel ausgewählt ist aus der Gruppe bestehend aus Thionylchlorid, Phosphoroxychlorid, Oxalylchlorid und Phosgen.

6. Verfahren nach einem der Ansprüche 1 bis 5, wobei das Chlorierungsmittel ausgewählt ist aus der Gruppe bestehend aus Thionylchlorid und Phosgen.

7. Verfahren nach einem der Ansprüche 1 bis 6, wobei das Chlorierungsmittel Thionylchlorid ist.

8. Verfahren nach einem der Ansprüche 1 bis 7, wobei die Base ausgewählt ist aus der Gruppe der schwachen Basen mit einem pK_{B}-Wert von grösser als 4,75.

9. Verfahren nach einem der Ansprüche 1 bis 8, wobei die Base ausgewählt ist aus der Gruppe der organische Amine.

10. Verfahren nach einem der Ansprüche 1 bis 9, wobei die Base ausgewählt ist aus der Gruppe bestehend aus Pyridin, 3-Methylpyridin und 3,5-Dimethylpyridin.

11. Verfahren nach einem der Ansprüche 1 bis 10, wobei das Lösungsmittel ausgewählt ist aus der Gruppe der aprotischen und dipolar aprotischen Lösungsmittel.

12. Verfahren nach einem der Ansprüche 1 bis 11, wobei das Lösungsmittel ausgewählt ist aus der Gruppe bestehend aus Ethylacetat, Propylacetat, Isopropylacetat, Butylacetat, Dichlormethan, Dichlorethan, Chlortoluol, Acetonitril, Butyronitril und Benzonitril.

13. Verfahren nach einem der Ansprüche 1 bis 12, wobei das Lösungsmittel ausgewählt ist aus der Gruppe bestehend aus Ethylacetat, Dichlormethan und Dichlorethan.

14. Verfahren nach einem der Ansprüche 1 bis 13, wobei die Cyanidquelle ausgewählt ist aus der Gruppe bestehend aus Acetoncyanhydrin und Alkalicyanide.

15. Verfahren nach einem der Ansprüche 1 bis 14, wobei die Cyanidquelle Acetoncyanhydrin ist.

## Claims

1. A process for the preparation of 3-alkylsulfinylbenzoyl derivatives of the formula (IIIa) by reaction of 3-alkylsulfinylbenzoic acids of the formula (Ib) with compounds of the formula (II) in the presence of a chlorinating agent and a base and optionally a cyanide source 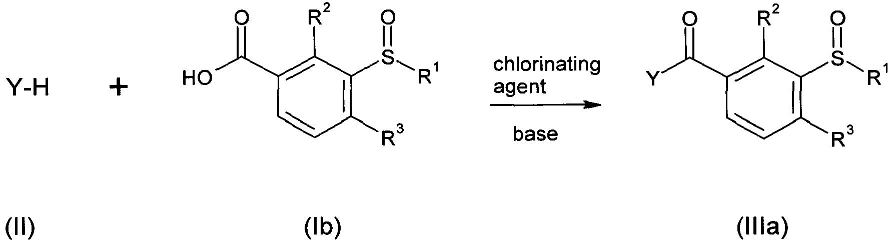 in which the radicals, symbols and indices have the following meanings:
R¹ is (C₁-C₈)-alkyl, (C₃-C₈)-cycloalkyl or benzyl substituted in each case by s radicals from the group consisting of halogen, (C₁-C₂)-alkoxy and halo-(C₁-C₂)-alkyl,
R² is hydrogen, mercapto, nitro, halogen, cyano, rhodano, (C₁-C₆)-alkyl, (C₁-C₆)-haloalkyl, (C₂-C₆)-alkenyl, (C₂-C₆)-haloalkenyl, (C₂-C₆)-alkynyl, (C₃-C₆)-haloalkynyl, (C₃-C₆)-cycloalkyl, OR⁴, OCOR⁴, OSO₂R⁴, S(O)ₙR⁴, SO₂OR⁴, SO₂N(R⁴)₂, NR⁴SO₂R⁴, NR⁴COR⁴, (C₁-C₆)-alkyl-S(O)ₙR⁴, (C₁-C₆)-alkyl-OR⁴, (C₁-C₆)-alkyl-OCOR⁴, (C₁-C₆)-alkyl-OSO₂R⁴, (C₁-C₆)-alkyl-SO₂OR⁴, (C₁-C₆)-alkyl-SO₂N(R⁴)₂ or (C₁-C₆)-alkyl-NR⁴COR⁴,
R³ is fluorine, chlorine, bromine, iodine, nitro, CF₃ or the group SO₂R,
R⁴ is hydrogen, (C₁-C₆)-alkyl, (C₂-C₆)-alkenyl, (C₂-C₆)-alkynyl, (C₃-C₆)-cycloalkyl, phenyl or phenyl-(C₁-C₆)-alkyl, where the six last-mentioned radicals are substituted by s radicals of the group hydroxy, mercapto, amino, cyano, nitro, rhodano, OR⁵, SR⁵, N(R⁵)₂, NOR⁵, OCOR⁵, SCOR⁵, NR⁵COR⁵, CO₂R⁵, COSR⁵, CON(R⁵)₂, (C₁-C₄)-alkyliminooxy, (C₁-C₄)-alkylcarbonyl, (C₁-C₄)-alkoxy-(C₂-C₆)-alkoxycarbonyl and (C₁-C₄)-alkylsulfonyl,
R⁵ is hydrogen, (C₁-C₆)-alkyl, (C₂-C₆)-alkenyl or (C₂-C₆)-alkynyl,
R is (C₁-C₄)-alkyl,
n is 0, 1 or 2,
s is 0, 1, 2 or 3,
Y is a radical Y1, Y2, Y3, Y4, Y5 or Y6: 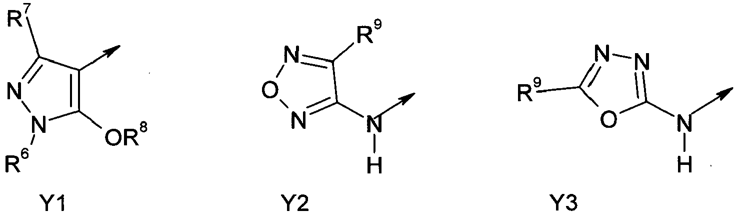 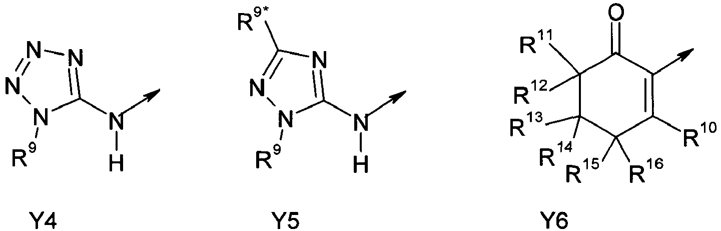
R⁶ is (C₁-C₄)-alkyl,
R⁷ is hydrogen or (C₁-C₄)-alkyl,
R⁸ is hydrogen, (C₁-C₆)-alkylsulfonyl, (C₁-C₄)-alkoxy-(C₁-C₆)-alkylsulfonyl, or phenylsulfonyl, thiophenyl-2-sulfonyl, benzoyl, benzoyl-(C₁-C₆)-alkyl or benzyl substituted in each case by s radicals from the group consisting of halogen, (C₁-C₄)-alkyl and (C₁-C₄)-alkoxy,
R⁹ is hydrogen, (C₁-C₆)-alkyl, (C₃-C₇)-cycloalkyl, halo-(C₁-C₆)-alkyl, (C₂-C₆)-alkenyl, halo-(C₂-C₆)-alkenyl, (C₂-C₆)-alkynyl, halo-(C₂-C₆)-alkynyl, CH₂R¹⁸ or phenyl substituted by s radicals from the group halogen, nitro, cyano, (C₁-C₆)-alkyl, halo-(C₁-C₆)-alkyl, (C₃-C₆)-cycloalkyl, S(O)ₙ-(C₁-C₆)-alkyl, (C₁-C₆)-alkoxy, halo-(C₁-C₆)-alkoxy, (C₁-C₆)-alkoxy-(C₁-C₄)-alkyl,
R^{9*} is hydrogen, nitro, halogen, cyano, rhodano, (C₁-C₆)-alkyl, halo-(C₁-C₆)-alkyl, (C₂-C₆)-alkenyl, halo-(C₂-C₆)-alkenyl, (C₂-C₆)-alkynyl, halo-(C₂-C₆)-alkynyl, (C₃-C₆)-cycloalkyl, (C₃-C₆)-cycloalkenyl, halo-(C₃-C₆)-cycloalkyl, (C₃-C₆)-cycloalkyl-(C₁-C₆)-alkyl, halo-(C₃-C₆)-cycloalkyl-(C₁-C₆)-alkyl, COR¹, COOR¹, OCOOR¹, NR¹COOR¹, C(O)N(R¹)₂, NR¹C(O)N(R¹)₂, OC(O)N(R¹)₂, CO(NOR¹)R¹, NR¹SO₂R², NR¹COR¹, OR¹, OSO₂R², S(O)ₙR², SO₂OR¹, SO₂N(R¹)₂, (C₁-C₆)-alkyl-S(O)ₙR², (C₁-C₆)-alkyl-OR¹, (C₁-C₆)-alkyl-OCOR¹, (C₁-C₆)-alkyl-OSO₂R², (C₁-C₆)-alkyl-CO₂R¹, (C₁-C₆)-alkyl-CN, (C₁-C₆)-alkyl-SO₂OR¹, (C₁-C₆)-alkyl-CON(R¹)₂, (C₁-C₆)-alkyl-SO₂N(R¹)₂, (C₁-C₆)-alkyl-NR¹COR¹, (C₁-C₆)-alkyl-NR¹SO₂R², N(R¹)₂, P(O)(OR⁵)₂, CH₂P(O)(OR⁵)₂, (C₁-C₆)-alkylphenyl, (C₁-C₆)-alkylheteroaryl, (C₁-C₆)-alkylheterocyclyl, phenyl, heteroaryl or heterocyclyl, where the last 6 radicals are substituted in each case by s radicals from the group halogen, nitro, cyano, (C₁-C₆)-alkyl, halo-(C₁-C₆)-alkyl, (C₃-C₆)-cycloalkyl, S(O)ₙ-(C₁-C₆)-alkyl, (C₁-C₆)-alkoxy, halo-(C₁-C₆)-alkoxy, (C₁-C₆)-alkoxy-(C₁-C₄)-alkyl and cyanomethyl, and where heterocyclyl carries 0 to 2 oxo groups,
R¹⁰ is hydroxy or SR¹⁷,
R¹¹ and R¹⁶, independently of one another, are hydrogen or (C₁-C₄)-alkyl,
or the radicals R¹¹ and R¹⁶ together form the unit Z, which is an oxygen atom or a sulfur atom or one to four methylene groups,
R¹² and R¹⁵, independently of one another, are hydrogen or (C₁-C₄)-alkyl,
R¹³ and R¹⁴, independently of one another, are hydrogen or (C₁-C₄)-alkyl or, together with the carbon atom to which they are bonded, form a carbonyl group,
R¹⁷ is (C₁-C₄)-alkyl, phenyl substituted by s radicals from the group consisting of nitro, cyano, (C₁-C₄)-alkyl, (C₁-C₄)-haloalkyl, (C₁-C₄)-alkoxy and (C₁-C₄)-haloalkoxy, or partially or completely halogenated phenyl,
R¹⁸ is acetoxy, acetamido, N-methylacetamido, benzoyloxy, benzamido, N-methylbenzamido, methoxycarbonyl, ethoxycarbonyl, benzoyl, methylcarbonyl, piperidinylcarbonyl, morpholinylcarbonyl, trifluoromethylcarbonyl, aminocarbonyl, methylaminocarbonyl, dimethylaminocarbonyl, (C₁-C₆)-alkoxy, (C₃-C₆)-cycloalkyl or phenyl substituted by s radicals from the group methyl, ethyl, methoxy, trifluoromethyl and halogen.

2. The process as claimed in claim 1, in which the radicals, symbols and indices have the following meanings:
R¹ is (C₁-C₄)-alkyl,
R² is nitro, halogen, (C₁-C₄)-alkyl, trifluoromethyl, (C₁-C₄)-alkoxy, methylsulfonyl, methoxymethyl, methoxymethoxymethyl, ethoxyethoxymethyl, ethoxymethoxymethyl, methoxyethoxymethyl, methoxypropoxymethyl, methylsulfonylmethyl, methylsulfonylethoxymethyl, methoxyethylsulfonylmethyl, methylsulfonylethylsulfonylmethyl,
R³ is fluorine, chlorine, bromine, iodine, CF₃ or the group SO₂R,
Y is a radical Y1, Y2, Y3, Y4, Y5 or Y6: 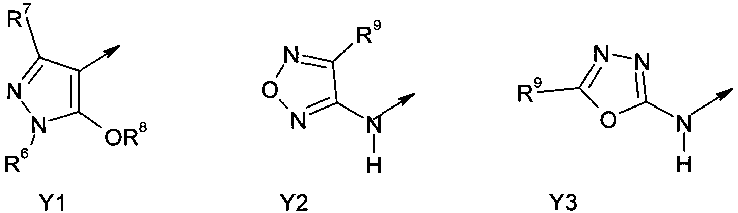 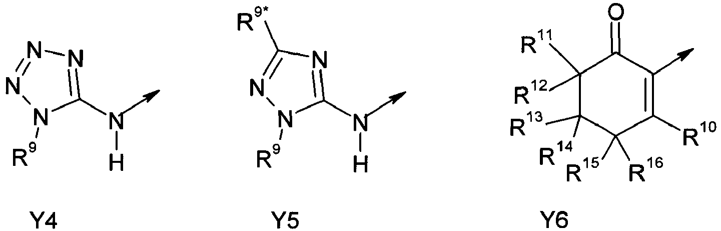
R⁶ is methyl, ethyl, n-propyl or isopropyl,
R⁷ is hydrogen, methyl, ethyl, n-propyl or isopropyl,
R⁸ is hydrogen, (C₁-C₃)-alkylsulfonyl, (C₁-C₂)-alkoxy-(C₁-C₄)-alkylsulfonyl, or phenylsulfonyl, thiophenyl-2-sulfonyl, benzoyl, benzoyl-(C₁-C₆)-alkyl or benzyl substituted in each case by s methyl groups,
s is 0, 1, 2 or 3,
R⁹ is (C₁-C₆)-alkyl, (C₃-C₇)-cycloalkyl, halo-(C₁-C₆)-alkyl, (C₃-C₇)-cycloalkylmethyl, methoxycarbonylmethyl, ethoxycarbonylmethyl, acetylmethyl, methoxymethyl, or phenyl or benzyl substituted by s radicals from the group methyl, methoxy, trifluoromethyl and halogen,
R^{9*} is hydrogen, (C₁-C₆)-alkyl, halo-(C₁-C₆)-alkyl, (C₃-C₆)-cycloalkyl, COR¹, OR¹, COOR¹ or (C₁-C₆)-alkylphenyl,
R¹⁰ is hydroxy,
R¹¹ and R¹⁶, independently of one another, are hydrogen or (C₁-C₄)-alkyl,
or the radicals R¹¹ and R¹⁶ together form a methylene group or ethylene group,
R¹² and R¹⁵, independently of one another, are hydrogen, methyl or ethyl,
R¹³ and R¹⁴, independently of one another, are hydrogen, methyl or ethyl.

3. The process as claimed in claim 1 or 2, where the reaction is carried out at a temperature of from -80 to -15°C.

4. The process as claimed in one of claims 1 to 3, where the reaction is carried out at a temperature of from -50 to -20°C.

5. The process as claimed in one of claims 1 to 4, where the chlorinating agent is selected from the group consisting of thionyl chloride, phosphorus oxychloride, oxalyl chloride and phosgene.

6. The process as claimed in one of claims 1 to 5, where the chlorinating agent is selected from the group consisting of thionyl chloride and phosgene.

7. The process as claimed in one of claims 1 to 6, where the chlorinating agent is thionyl chloride.

8. The process as claimed in one of claims 1 to 7, where the base is selected from the group of weak bases with a pK_{B} value greater than 4.75.

9. The process as claimed in one of claims 1 to 8, where the base is selected from the group of organic amines.

10. The process as claimed in one of claims 1 to 9, where the base is selected from the group consisting of pyridine, 3-methylpyridine and 3,5-dimethylpyridine.

11. The process as claimed in one of claims 1 to 10, where the solvent is selected from the group of aprotic and dipolar aprotic solvents.

12. The process as claimed in one of claims 1 to 11, where the solvent is selected from the group consisting of ethyl acetate, propyl acetate, isopropyl acetate, butyl acetate, dichloromethane, dichloroethane, chlorotoluene, acetonitrile, butyronitrile and benzonitrile.

13. The process as claimed in one of claims 1 to 12, where the solvent is selected from the group consisting of ethyl acetate, dichloromethane and dichloroethane.

14. The process as claimed in one of claims 1 to 13, where the cyanide source is selected from the group consisting of acetone cyanohydrin and alkali metal cyanides.

15. The process as claimed in one of claims 1 to 14, where the cyanide source is acetone cyanohydrin.

## Revendications

1. Procédé pour la préparation de dérivés 3-alkylsulfinylbenzoyle de formule (IIIa) par réaction d'acides 3-alkylsulfinylbenzoïques de formule (Ib) avec des composés de formule (II) en présence d'un agent de chloration et d'une base et le cas échéant dune source de cyanure 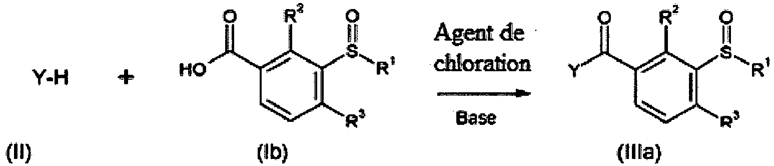 dans lesquelles les radicaux, les symboles et les indices présentent les significations suivantes :
R¹ signifie (C₁-C₈)-alkyle, (C₃-C₈)-cycloalkyle ou benzyle à chaque fois substitué par s radicaux du groupe constitué par halogéno, (C₁-C₂)-alcoxy et halogéno-(C₁-C₂)-alkyle,
R² signifie hydrogène, mercapto, nitro, halogéno, cyano, thiocyano, (C₁-C₆)-alkyle, (C₁-C₆)-halogénoalkyle, (C₂-C₆)-alcényle, (C₂-C₆)-halogénoalcényle, (C₂-C₆)-alcynyle, (C₃-C₆)-halogénoalcynyle, (C₃-C₆)-cycloalkyle, OR⁴, OCOR⁴, OSO₂R⁴, S(O)ₙR⁴, SO₂OR⁴, SO₂N(R⁴)₂, NR⁴SO₂R⁴, NR⁴COR⁴, (C₁-C₆)-alkyl-S(O)ₙR⁴, (C₁-C₆)-alkyl-OR⁴, (C₁-C₆)-alkyl-OCOR⁴, (C₁-C₆) -alkyl-OSO₂R⁴, (C₁-C₆)-alkyl-SO₂OR⁴, (C₁-C₆)-alkyl-SO₂N(R⁴)₂ ou (C₁-C₆)-alkyl-NR⁴COR⁴,
R³ signifie fluor, chlore, brome, iode, nitro, CF₃ ou le groupe SO₂R,
R⁴ signifie hydrogène, (C₁-C₆)-alkyle, (C₂-C₆)-alcényle, (C₂-C₆)-alcynyle, (C₃-C₆)-cycloalkyle, phényle ou phényl-(C₁-C₆)-alkyle, les six derniers radicaux mentionnés étant substitués par s radicaux du groupe constitué par hydroxy, mercapto, amino, cyano, nitro, thiocyano, OR⁵, SR⁵, N(R⁵)₂, NOR⁵, OCOR⁵, SCOR⁵, NR⁵COR⁵, CO₂R⁵, COSR⁵, CON(R⁵)₂, (C₁-C₄)-alkyliminooxy, (C₁-C₄)-alkylcarbonyle, (C₁-C₄)-alcoxy-(C₂-C₆)-alcoxycarbonyle et (C₁-C₄)-alkylsulfonyle,
R⁵ signifie hydrogène, (C₁-C₆)-alkyle, (C₂-C₆)-alcényle ou (C₂-C₆)-alcynyle,
R signifie (C₁-C₄)-alkyle,
n signifie 0, 1 ou 2,
s signifie 0, 1, 2 ou 3,
Y signifie un radical Y1, Y2, Y3, Y4, Y5 ou Y6, 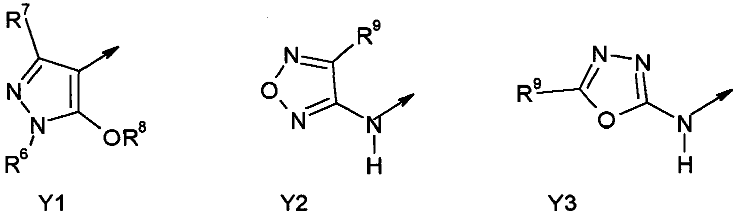 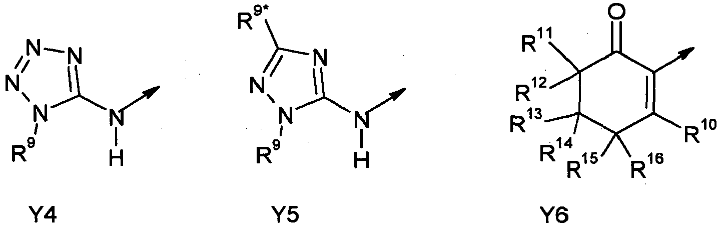
R⁶ signifie (C₁-C₄)-alkyle,
R⁷ signifie hydrogène ou (C₁-C₄)-alkyle,
R⁸ signifie hydrogène, (C₁-C₆)-alkylsulfonyle, (C₁-C₄)-alcoxy-(C₁-C₆)-alkylsulfonyle ; ou phénylsulfonyle, thiophényl-2-sulfonyle, benzoyle, benzoyl-(C₁-C₆)-alkyle ou benzyle à chaque fois substitué par s radicaux du groupe constitué par halogéno, (C₁-C₄)-alkyle et (C₁-C₄)-alcoxy,
R⁹ signifie hydrogène, (C₁-C₆)-alkyle, (C₃-C₇)-cycloalkyle, halogéno-(C₁-C₆)-alkyle, (C₂-C₆)-alcényle, halogéno-(C₂-C₆)-alcényle, (C₂-C₆)-alcynyle, halogéno-(C₂-C₆)-alcynyle, CH₂R¹⁸ ; ou phényle substitué par s radicaux du groupe constitué par halogéno, nitro, cyano, (C₁-C₆)-alkyle, halogéno-(C₁-C₆)-alkyle, (C₃-C₆)-cycloalkyle, S(O)ₙ-(C₁-C₆)-alkyle, (C₁-C₆)-alcoxy, halogéno-(C₁-C₆)-alcoxy, (C₁-C₆)-alcoxy-(C₁-C₄)-alkyle,
R^{9*} signifie hydrogène, nitro, halogéno, cyano, thiocyano, (C₁-C₆)-alkyle, halogéno-(C₁-C₆)-alkyle, (C₂-C₆)-alcényle, halogéno-(C₂-C₆)-alcényle, (C₂-C₆)-alcynyle, halogéno-(C₂-C₆)-alcynyle, (C₃-C₆)-cycloalkyle, (C₃-C₆)-cycloalcényle, halogéno-(C₃-C₆)-cycloalkyle, (C₃-C₆)-cycloalkyl-(C₁-C₆)-alkyle, halogéno-(C₃-C₆)-cycloalkyl-(C₁-C₆)-alkyle, COR¹, COOR¹, OCOOR¹, NR¹COOR¹, C(O)N(R¹)₂, NR¹C(O)N(R¹)₂, OC(O)N(R¹)₂, CO(NOR¹)R¹, NR¹SO₂R², NR¹COR¹, OR¹, OSO₂R², S(O)ₙR², SO₂OR¹, SO₂N(R¹)₂, (C₁-C₆)-alkyl-S(O)ₙR², (C₁-C₆) -alkyl-OR¹, (C₁-C₆)-alkyl-OCOR¹, (C₁-C₆)-alkyl-OSO₂R², (C₁-C₆)-alkyl-CO₂R¹, (C₁-C₆)-alkyl-CN, (C₁-C₆)-alkyl-SO₂OR¹, (C₁-C₆)-alkyl-CON(R¹)₂, (C₁-C₆)-alkyl-SO₂N(R¹)₂, (C₁-C₆)-alkyl-NR¹COR¹, (C₁-C₆)-alkyl-NR¹SO₂R², N(R¹)₂, P(O)(OR⁵)₂, CH₂P(O)(OR⁵)₂, (C₁-C₆)-alkyl-phényle, (C₁-C₆)-alkyl-hétéroaryle, (C₁-C₆)-alkyl-hétérocyclyle, phényle, hétéroaryle ou hétérocyclyle, les 6 derniers radicaux étant à chaque fois substitués par s radicaux du groupe constitué par halogéno, nitro, cyano, (C₁-C₆)-alkyle, halogéno-(C₁-C₆)-alkyle, (C₃-C₆)-cycloalkyle, S(O)ₙ-(C₁-C₆)-alkyle, (C₁-C₆)-alcoxy, halogéno-(C₁-C₆)-alcoxy, (C₁-C₆)-alcoxy-(C₁-C₄)-alkyle et cyanométhyle et hétérocyclyle portant 0 à 2 groupes oxo,
R¹⁰ signifie hydroxy ou SR¹⁷,
R¹¹ et R¹⁶ signifient, indépendamment l'un de l'autre, hydrogène ou (C₁-C₄) -alkyle, ou les radicaux R¹¹ et R¹⁶ forment ensemble l'unité Z, qui représente un atome d'oxygène ou de soufre ou un à quatre groupes méthylène,
R¹² et R¹⁵ signifient, indépendamment l'un de l'autre, hydrogène ou (C₁-C₄) -alkyle,
R¹³ et R¹⁴ signifient, indépendamment l'un de l'autre, hydrogène ou (C₁-C₄)-alkyle ou forment ensemble avec l'atome de carbone auquel ils sont liés un groupe carbonyle,
R¹⁷ signifie (C₁-C₄)-alkyle ; phényle substitué par s radicaux du groupe constitué par nitro, cyano, (C₁-C₄)-alkyle, (C₁-C₄)-halogénoalkyle, (C₁-C₄)-alcoxy et (C₁-C₄)-halogénoalcoxy ; ou phényle partiellement ou complètement halogéné,
R¹⁸ signifie acétoxy, acétamido, N-méthylacétamido, benzoyloxy, benzamido, N-méthylbenzamido, méthoxycarbonyle, éthoxycarbonyle, benzoyle, méthylcarbonyle, pipéridinylcarbonyle, morpholinylcarbonyle, trifluorométhylcarbonyle, aminocarbonyle, méthylaminocarbonyle, diméthylaminocarbonyle, (C₁-C₆)-alcoxy, (C₃-C₆)-cycloalkyle ; ou phényle substitué par s radicaux du groupe constitué par méthyle, éthyle, méthoxy, trifluorométhyle et halogéno.

2. Procédé selon la revendication 1, dans lequel les radicaux, les symboles et les indices présentent les significations suivantes :
R¹ signifie (C₁-C₄)-alkyle,
R² signifie nitro, halogéno, (C₁-C₄)-alkyle, trifluorométhyle, (C₁-C₄)-alcoxy, méthylsulfonyle, méthoxyméthyle, méthoxyméthoxyméthyle, éthoxyéthoxyméthyle, éthoxyméthoxyméthyle, méthoxyéthoxyméthyle, méthoxypropoxyméthyle, méthylsulfonylméthyle, méthylsulfonyléthoxyméthyle, méthoxyéthylsulfonylméthyle, méthylsulfonyléthylsulfonylméthyle,
R³ signifie fluor, chlore, brome, iode, CF₃ ou le groupe SO₂R,
Y signifie un radical Y1, Y2, Y3, Y4, Y5 ou Y6, 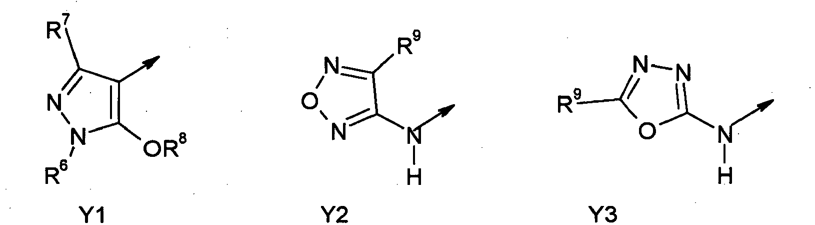 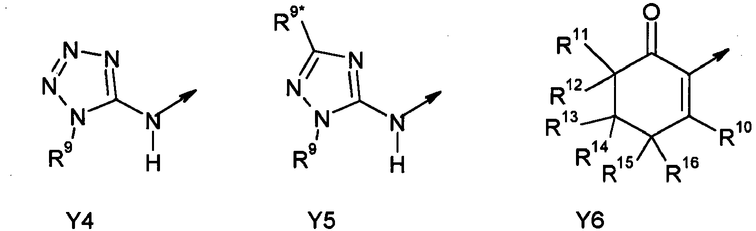
R⁶ signifie méthyle, éthyle, n-propyle, ou i-propyle,
R⁷ signifie hydrogène, méthyle, éthyle, n-propyle ou i-propyle,
R⁸ signifie hydrogène, (C₁-C₃)-alkylsulfonyle, (C₁-C₂)-alcoxy-(C₁-C₄)-alkylsulfonyle ; ou phénylsulfonyle, thiophényl-2-sulfonyle, benzoyle, benzoyl-(C₁-C₆)-alkyle ou benzyle à chaque fois substitué par s groupes méthyle,
s signifie 0, 1, 2 ou 3,
R⁹ signifie (C₁-C₆)-alkyle, (C₃-C₇)-cycloalkyle, halogéno-(C₁-C₆)-alkyle, (C₃-C₇)-cycloalkylméthyle, méthoxycarbonylméthyle, éthoxycarbonylméthyle, acétylméthyle, méthoxyméthyle ; ou phényle ou benzyle substitués par s radicaux du groupe constitué par méthyle, méthoxy, trifluorométhyle et halogéno,
R^{9*} signifie hydrogène, (C₁-C₆)-alkyle, halogéno-(C₁-C₆)-alkyle, (C₃-C₆)-cycloalkyle, COR¹, OR¹, COOR¹ ou (C₁-C₆)-alkyl-phényle,
R¹⁰ signifie hydroxy,
R¹¹ et R¹⁶ signifient, indépendamment l'un de l'autre, hydrogène ou (C₁-C₄)-alkyle, ou les radicaux R¹¹ et R¹⁶ forment ensemble un groupe méthylène ou éthylène,
R¹² et R¹⁵ signifient, indépendamment l'un de l'autre, hydrogène, méthyle ou éthyle,
R¹³ et R¹⁴ signifient, indépendamment l'un de l'autre, hydrogène, méthyle ou éthyle.

3. Procédé selon la revendication 1 ou 2, la réaction étant réalisée à une température de -80 à -15°C.

4. Procédé selon l'une quelconque des revendications 1 à 3, la réaction étant réalisée à une température de -50 à -20°C.

5. Procédé selon l'une quelconque des revendications 1 à 4, l'agent de chloration étant choisi dans le groupe constitué par le chlorure de thionyle, l'oxychlorure de phosphore, le chlorure d'oxalyle et le phosgène.

6. Procédé selon l'une quelconque des revendications 1 à 5, l'agent de chloration étant choisi dans le groupe constitué par le chlorure de thionyle et le phosgène.

7. Procédé selon l'une quelconque des revendications 1 à 6, l'agent de chloration étant le chlorure de thionyle.

8. Procédé selon l'une quelconque des revendications 1 à 7, la base étant choisie dans le groupe des bases faibles présentant un pK_{B} supérieur à 4,75.

9. Procédé selon l'une quelconque des revendications 1 à 8, la base étant choisie dans le groupe des amines organiques.

10. Procédé selon l'une quelconque des revendications 1 à 9, la base étant choisie dans le groupe constitué par la pyridine, la 3-méthylpyridine et la 3,5-diméthylpyridine.

11. Procédé selon l'une quelconque des revendications 1 à 10, le solvant étant choisi dans le groupe des solvants aprotiques et aprotiques dipolaires.

12. Procédé selon l'une quelconque des revendications 1 à 11, le solvant étant choisi dans le groupe constitué par l'acétate d'éthyle, l'acétate de propyle, l'acétate d'isopropyle, l'acétate de butyle, le dichlorométhane, le dichloroéthane, le chlorotoluène, l'acétonitrile, le butyronitrile et le benzonitrile.

13. Procédé selon l'une quelconque des revendications 1 à 12, le solvant étant choisi dans le groupe constitué par l'acétate d'éthyle, le dichlorométhane et le dichloroéthane.

14. Procédé selon l'une quelconque des revendications 1 à 13, la source de cyanure étant choisie dans le groupe constitué par l'acétonecyanhydrine et les cyanures alcalins.

15. Procédé selon l'une quelconque des revendications 1 à 14, la source de cyanure étant l'acétonecyanhydrine.
